(19) **Europäisches Patentamt / European Patent Office / Office européen des brevets**

(11) **EP 2 163 264 B1**

(12) # FASCICULE DE BREVET EUROPEEN

(45) Date de publication et mention
de la délivrance du brevet:
**25.04.2012 Bulletin 2012/17**

(51) Int Cl.:
***A61L 9/12*** *(2006.01)*

(21) Numéro de dépôt: **09009819.5**

(22) Date de dépôt: **29.07.2009**

(54) **Procédé de diffusion d'une stubstance volatile et dispositif mettant en oeuvre un tel procédé**

Diffusionsverfahren einer flüchtigen Substanz und Vorrichtung zur Umsetzung eines solchen Verfahrens

Method for distributing a volatile substance and device implementing such a method

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **28.08.2008 FR 0804739**

(43) Date de publication de la demande:
**17.03.2010 Bulletin 2010/11**

(73) Titulaires:
- **M. Lenfant, Jean-Pierre**
  **87600 Rochechouart (FR)**
- **Hiblot, Norbert**
  **56000 Vannes (FR)**

(72) Inventeur: **Lenfant, Jean-Pierre**
**87600 Rochechouart (FR)**

(74) Mandataire: **Demulsant, Xavier**
**Dejade & Biset**
**35, rue de Châteaudun**
**75009 Paris (FR)**

(56) Documents cités:
**WO-A-03/019082     US-A- 5 032 230**
**US-A- 5 143 607     US-A1- 2006 145 368**

Printed by Jouve, 75001 PARIS (FR)

**Description**

L'invention a trait au domaine de la diffusion des substances volatiles telles que les parfums d'ambiance. Plus précisément, l'invention se rapporte au domaine de la diffusion d'une telle substance dans un local, tel qu'un salon, un bureau, une chambre ou une salle de réunion, ou même dans l'habitacle d'un véhicule.

La diffusion de la substance se fait de manière naturelle par le processus d'évaporation des espèces volatiles composant la substance, généralement sous forme liquide, lesquelles sont ensuite transportées par des mouvements d'air. Toutefois, la diffusion naturelle se révèle peu adaptée dans les cas où le volume du local dans lequel on souhaite diffuser est grand, et notamment dans le cas de la diffusion de parfum. En effet, par le processus naturel, les espèces évaporées ont tendance à stationner autour de la source, les mouvements d'air étant insuffisants pour disséminer les espèces dans l'ensemble du local. C'est pourquoi il est nécessaire d'utiliser un diffuseur mécanique.

Les diffuseurs mécaniques, notamment de parfum, sont largement utilisés dans ce but. Une de leurs fonctions premières est de masquer des odeurs indésirables en les plaçant à proximité d'endroits malodorants. Par ailleurs, un diffuseur de parfum peut également être utilisé afin d'apporter une note d'ambiance dans une pièce. Ainsi, dans un lieu d'habitation par exemple, on aura avantage à choisir un parfum chaleureux pour une pièce à vivre (salon, chambre), et a contrario un parfum plus frais pour une salle d'eau.

Les entreprises utilisent elles aussi, de plus en plus, des diffuseurs de parfum. En effet, il peut être agréable pour un client d'être reçu dans une salle d'attente parfumée. En outre, une entreprise peut se servir d'un même parfum pour l'ensemble de ses locaux, situés à des adresses différentes, en tant que signe distinctif, au même titre qu'un logo ou qu'une police de couleurs.

D'autres substances sont également utilisées pour être diffusées, par exemple les désinfectants pour des environnements hospitaliers, des agents absorbeurs d'odeur ou masquant les odeurs.

Un diffuseur comprend en général un flacon dans lequel est stockée la substance, de préférence, sous forme liquide. Une partie de la substance est prélevée afin d'être évaporée et disséminée. Afin d'obtenir une diffusion optimale dans un local, qui peut être de volume important, la substance est généralement diffusée au moyen d'un générateur de débit d'air, qui entraîne le parfum dans le local.

Le débit d'air peut être directement amené sur la substance dans le flacon, les espèces à la surface étant alors transportées par le débit d'air. Le document JP 2002-85542 présente un tel diffuseur, dans lequel un flacon contient la substance sous forme liquide. Un conduit, relié par une extrémité à une source d'un débit de gaz, est inséré dans le flacon, et son extrémité libre est immergée dans la substance. Un deuxième conduit par lequel s'échappe le parfum mélangé au gaz relie l'intérieur du flacon à une veine d'air, assurant la dissémination dans une pièce.

En variante, c'est la substance qui est amenée dans le débit d'air. Un exemple d'un tel diffuseur est décrit dans le document FR 2 657 019. La substance est contenue sous forme liquide dans un flacon, dans lequel plonge un conduit. L'extrémité supérieure de ce conduit est rétrécie et est placée dans une veine d'air. La substance est aspirée dans le conduit par effet Venturi jusqu'à la section rétrécie, où elle se vaporise et est diffusée à l'air.

La substance peut également être amenée dans le débit d'air par un matériau absorbant, telle qu'une mèche. Un exemple est donné dans le document WO2007128148. La mèche est placée dans le flacon, la base de la mèche étant immergée dans la substance liquide et le sommet étant placé dans le débit d'air. Le liquide remonte depuis la base de la mèche jusqu'au sommet par capillarité, puis les espèces volatiles sont arrachées par le débit d'air, les emportant dans le local à parfumer.

Un des inconvénients de tels diffuseurs est qu'ils ne se vident pas complètement. En effet, les substances volatiles comprennent généralement des espèces se comportant différemment vis-à-vis du phénomène de diffusion. Ce comportement peut être quantifié au moyen de la pression de vapeur saturante.

La pression de vapeur saturante est la pression à laquelle un composé en phase gazeuse est à l'équilibre avec sa phase liquide. Plus cette pression est élevée pour un composé, plus le composé est dit léger, c'est-à-dire qu'il est plus volatil, et est diffusé facilement dans l'air.

En général, la pression de vapeur saturante des espèces d'une substance volatile telle qu'un parfum peut varier dans un rapport de 1 à 100. De ce fait, les espèces les plus volatiles sont diffusées aisément, tandis que les autres, plus lourdes, ne peuvent ni être entrainées par le débit d'air, ni remonter par effet Venturi ou par capillarité, et restent au fond du flacon, laissant à un utilisateur un sentiment de gaspillage.

Afin de remédier à ce problème, le document EP 1 252 900 présente un diffuseur de parfum dans lequel un parfum sous forme liquide est pompé puis amené au moyen d'un conduit sur un élément absorbant, afin d'augmenter la surface de diffusion. L'élément étant placé dans une veine d'air, le parfum est diffusé à l'air. Une gouttière disposée à la place de l'élément absorbant permet de récupérer les composants les plus lourds du parfum qui ne peuvent être diffusés et qui s'écoulent par gravité. Les composants non diffusés étant récupérés, le flacon pourra être totalement vidé.

Toutefois, le dispositif se révèle délicat à mettre en place et fastidieux à entretenir, à cause de la présence d'un réservoir de récupération et de moyens de pilotage électriques.

Dans le document FR 2 797 189, l'ouverture du flacon est dirigée vers le bas, ce qui permet d'utiliser la totalité du parfum liquide contenu dans ce flacon. Une canne aspirante placée dans l'ouverture permet de prélever du parfum et de l'amener par capillarité sur les pales d'un ventilateur, lesquelles sont recouvertes d'un disque poreux. Le parfum est alors projeté par centrifugation.

Le contact entre le parfum et le ventilateur, ainsi que les pièces environnantes ayant une fonction mécanique sur lesquelles du parfum sera éventuellement projeté, peut se révéler nocif pour ces pièces. Effectivement, il est connu que certaines substances utilisées pour être diffusées, et notamment dans les parfums, sont corrosives vis-à-vis des pièces mécaniques métalliques composant le dispositif de diffusion, entrainant une usure prématurée des pièces. D'autres pièces subissant des contraintes mécaniques, par exemple les tuyaux en plastique souple dans lesquels circule le parfum, sont susceptibles de s'user plus rapidement à cause d'éventuelles réactions avec le parfum. Dès lors, il est nécessaire de mettre en place un système de protection de ces pièces, engendrant des coûts supplémentaires.

La présente invention vise notamment à apporter une solution aux divers problèmes mentionnés ci-dessus

Un premier objet de l'invention est de proposer un procédé de diffusion d'une substance volatile, tel qu'un parfum, dans lequel la diffusion est assurée aussi bien pour les espèces à pression de vapeur saturante élevée que pour les espèces à pression de vapeur saturante plus faible.

Un second objet de l'invention est de proposer un diffuseur permettant la mise en oeuvre du procédé, et limitant tout contact entre des pièces mécaniques environnantes et la substance, optimisant la diffusion.

Selon un premier aspect, l'invention propose un procédé de diffusion d'une substance volatile dans un local, la substance étant contenue dans un réservoir et comprenant des espèces présentant des pressions de vapeur saturante différentes, le procédé comprenant les étapes consistant à :

- générer un débit d'air ;
- introduire au moins une partie du débit d'air dans une chambre contenant un mélange d'air et d'espèces volatiles ;
- diffuser le mélange dans l'atmosphère,

le débit d'air introduit dans la chambre de mélange étant ajusté en fonction du volume de substance contenue dans le réservoir.

De cette façon, le débit d'air est adapté au contenu du réservoir et aux valeurs de pression de vapeur saturante des espèces présentes dans le réservoir.

Selon un mode de réalisation préféré, le débit d'air introduit dans la chambre de mélange est réajusté à la hausse avec la diminution du volume de substance dans le réservoir. Dès lors, les espèces ayant une pression de vapeur saturante plus élevée et donc plus difficiles à diffuser sont emportées par un débit d'air plus important.

Selon un deuxième aspect, l'invention propose un dispositif de diffusion d'une substance volatile, ce dispositif comportant :

- un générateur d'un débit d'air ;
- un réservoir contenant la substance ;
- une chambre contenant un mélange d'air et d'espèces issues de l'évaporation de la substance ;
- un conduit d'amenée d'air mettant en communication le débit d'air avec la chambre, le conduit présentant une surface d'échange d'air avec la chambre de mélange ;
- une évacuation mettant la chambre de mélange en communication avec l'atmosphère,

ce dispositif comprenant des moyens d'ajustement de la surface d'échange en fonction du niveau de substance dans le réservoir, permettant la mise en oeuvre du procédé décrit ci-dessus.

La surface d'échange comprend par exemple au moins une ouverture latérale ménagée dans le conduit et débouchant au moins partiellement dans la chambre de mélange. Une partie du débit d'air est donc amenée au dessus de la substance, tandis que lorsque le niveau de la substance sera suffisamment faible pour dévoiler l'extrémité du conduit dans le réservoir, la surface d'échange d'air sera augmentée.

Selon un premier mode de réalisation, plusieurs ouvertures latérales dont l'une au moins débouche dans la chambre de mélange peuvent être réparties sur le conduit, de sorte que plus le niveau de la substance dans le réservoir baisse, plus la surface d'échange d'air augmente.

Les ouvertures sont par exemple circulaires.

Le diamètre des ouvertures peut être croissant en direction d'une extrémité libre aval du conduit, le débit d'air sortant à proximité de la surface de la substance dans le réservoir étant alors de plus en plus grand, favorisant la diffusion des espèces les moins volatiles.

Selon un deuxième mode de réalisation le conduit comprend une unique ouverture latérale sous forme d'une découpe allongée, l'augmentation de la surface d'échange d'air se faisant de manière continue.

La découpe peut aller en s'évasant depuis la chambre de mélange vers une extrémité libre aval du conduit, afin d'augmenter le débit d'air sur la surface de la substance dans le réservoir au fur et à mesure que le niveau baisse.

Par ailleurs, un solide poreux peut être introduit dans le réservoir, ce solide se trouvant alors en contact avec la substance pour absorber une quantité de la substance. La surface d'évaporation des espèces de la substance se trouve alors augmentée.

D'autres objets et avantages de l'invention apparaîtront à la lumière de la description faite ci-après en référence aux dessins annexés dans lesquels :

- la figure 1 est une vue en coupe d'un dispositif de diffusion, comprenant trois réservoirs d'une substance volatile, les réservoirs étant munis d'un conduit amenant un débit d'air dans le réservoir et d'une conduite de sortie à l'atmosphère.

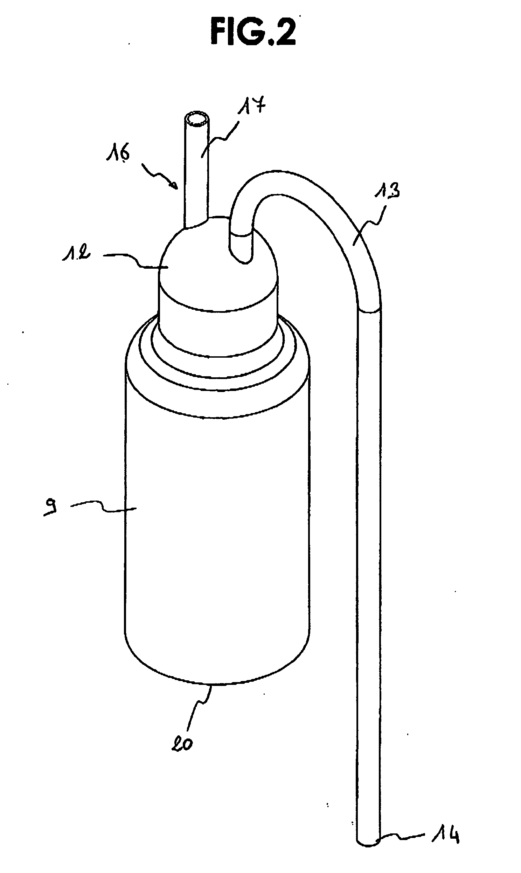

- la figure 2 est une vue en perspective d'un réservoir contenant la substance volatile et muni d'un couvercle ;
- la figure 3 est une vue en coupe de la figure 1 selon un premier mode de réalisation ;
- la figure 4 est une vue en coupe de la figure 1 selon un deuxième mode de réalisation ;
- la figure 5 est une vue en coupe de la figure 1 selon un troisième mode de réalisation ;
- la figure 6 est une vue en coupe de la figure 1 selon un quatrième mode de réalisation ;

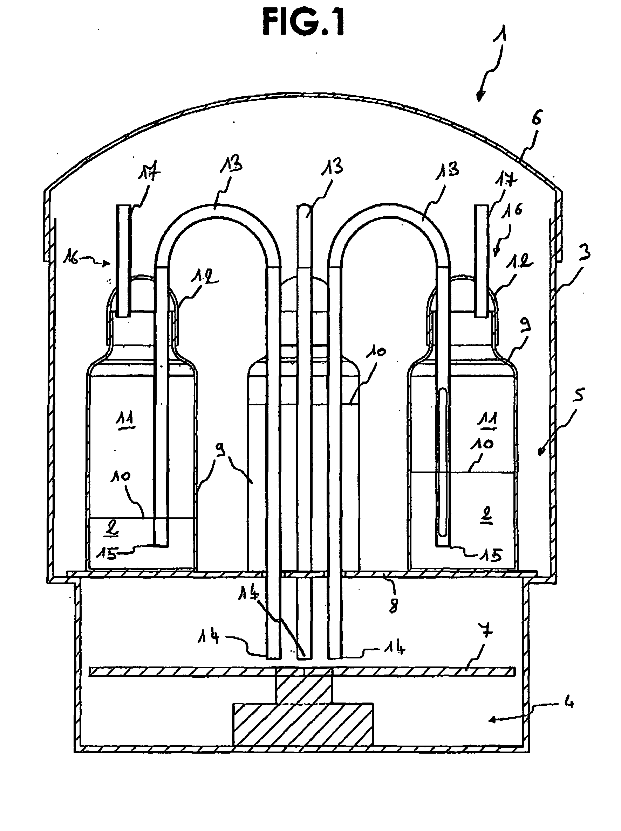

Sur la figure 1 est représenté un dispositif 1 de diffusion d'une substance 2 volatile.

Par substance volatile, on désigne ici toute substance comportant des espèces aptes à s'évaporer par les surfaces libres de la substance.

Le dispositif 1 comprend un bâti 3 séparé en deux parties : une partie 4 inférieure et une partie 5 supérieure, cette dernière étant fermée au moyen d'un couvercle 6 amovible percé d'ouvertures pour la diffusion du parfum. Un générateur 7 d'un débit d'air, tel qu'un ventilateur électrique, est placé dans la partie 4 inférieure, de sorte à diriger le débit d'air vers la partie 5 supérieure.

La partie 4 inférieure et la partie 5 supérieure sont séparées par une grille 8, laissant passer le débit d'air de la partie 4 inférieure vers la partie 5 supérieure.

Sur la grille 8, entre les deux parties 4 et 5 du bâti 3 est posé au moins un flacon 9 formant un réservoir contenant la substance 2 volatile.

La substance 2 est de préférence sous forme liquide, et présente une surface 10 supérieure libre. Le flacon 9 n'est pas rempli complètement, de sorte qu'il définit au dessus de la substance 2 une chambre 11 de mélange air-parfum. Le réservoir 9 est fermé à son sommet par un couvercle 12, afin de ne pas laisser s'échapper les espèces évaporées en raison de mouvements d'air non contrôlés.

Un conduit 13 coudé en forme de canne est placé entre le débit d'air et la substance 2. Ce conduit présente une extrémité 14 inférieure ou amont, ouverte, débouchant dans le débit d'air, préférentiellement dans la partie 4 inférieure au plus près du générateur 7, et une extrémité 15 supérieure ou aval, débouchant dans le flacon 9 pour y amener le débit d'air. Plus précisément, l'extrémité 15 aval débouche dans la chambre 11 ou, de préférence, directement dans la substance 2. Le conduit 13 coudé est de préférence réalisé dans une matière, telle que l'inox ou le verre, inerte vis-à-vis de la substance 2 dans laquelle il est immergé.

Une évacuation 16 permet de mettre en communication la chambre 11 de mélange avec l'atmosphère d'un local dans lequel la substance 2 doit être diffusée. Cette évacuation 16 est de préférence formée par un deuxième conduit 17 traversant le couvercle 12 et présentant une extrémité inférieure débouchant dans la chambre 11 de mélange, et une extrémité supérieure débouchant dans la partie 4 supérieure du bâti 3.

Le conduit 13 amenant le débit d'air dans le réservoir 9 et le conduit 17 de sortie sont avantageusement introduits dans le flacon 9 au moyen d'ouvertures pratiquées dans le couvercle 12.

La substance 2 volatile est par exemple un parfum, contenu dans des huiles essentielles, extraites de matières naturelles ou résultant d'une synthèse. Il comprend des espèces ayant des pressions de vapeur saturante différentes, mélangées dans un solvant liquide également volatil.

Lorsque le générateur 7 est mis en route, une partie du débit d'air est introduite dans le flacon 9 par le conduit 13 coudé. Les espèces volatiles à la surface 10 libre de la substance 2 sont évaporées et transportées dans la chambre 11 de mélange par un phénomène de brassage dû au débit d'air. La pression dans la chambre 11 de mélange augmente, la surpression créant ainsi un flux d'air au travers de l'évacuation 16. Une partie du débit d'air en provenance du générateur 7 atteignant le sommet du réservoir 9, les espèces volatiles sortant du flacon 9 par le conduit 17 sont entraînées par ce débit et diffusent dans l'atmosphère au travers du couvercle 6 du bâti 3.

Le générateur 7. aspire de l'air dans l'atmosphère par une entrée d'air pratiquée dans la partie 4 inférieure du bâti 3. Afin de limiter la dispersion de particules de poussière se trouvant dans l'atmosphère par le dispositif 1, un média filtrant peut être placé sur l'entrée d'air, de sorte que ces particules ne soient pas aspirées par le générateur 7.

Les inventeurs ont observé que la différence de pression de vapeur saturante entre les espèces engendre une évaporation inégale : les espèces les plus volatiles sont emportées par le débit d'air tandis que les autres restent au fond du flacon 9. Dès lors, passé un certain temps, les espèces les plus volatiles ayant été évaporées puis diffusées vers l'atmosphère, le débit d'air n'est plus assez fort pour générer un brassage efficace à la surface 10 libre et entraîner les espèces les moins volatiles, qui stagnent alors dans le flacon 9.

C'est pourquoi il est prévu d'ajuster le débit d'air en fonction du volume de la substance 2 restant dans le flacon 9. En effet, alors que les espèces les plus volatiles sont évaporées et diffusées progressivement, le volume diminue et le niveau de la surface 10 libre baisse. Pour entrainer les espèces plus lourdes, il convient alors d'augmenter le débit d'air afin d'accentuer le phénomène de brassage et de les transporter à leur tour. Dès lors, l'augmentation du débit d'air amené à la surface 10 libre de la substance 2 dans le flacon 9 au fur et à mesure que le volume diminue permet de transporter la totalité des espèces volatiles de la substance 2.

L'exemple qui suit donne un exemple du calcul pour l'ajustement du débit d'air dans le flacon 9, pour le cas où la substance 2 à diffuser est un parfum.

On part de l'hypothèse que le local dans lequel on souhaite diffuser une substance volatile est d'un volume de 300 m³, l'air y étant renouvelé cinq fois par heure. Il convient alors de choisir un ventilateur générant un débit d'air de 1500 m³ par heure.

En moyenne, le taux de substance volatile, c'est-à-dire d'espèces composant la substance, dans l'air ambiant dans le cas d'un parfum doit être compris entre 0,5 et 1 mg par m³ d'air. En prenant un taux médian de 0,75 mg par m³ d'air, il faut donc 300 x 0,75 = 225 mg de parfum pour l'ensemble du local, soit 5 x 225 = 1125 mg de parfum par heure pour le local. À supposer que le système fonctionne 12 heures par jours, et que l'on souhaite maintenir ce fonctionnement pendant un mois (30 jours), la quantité de parfum à prévoir est par conséquent de 405 g.

L'éthanol est souvent pris comme solvant et produit de base des parfums. Il a une pression de vapeur saturante de 40 mmHg à 20°C, une masse molaire de 46 g.mol⁻¹ et un volume molaire de 22,4 l.mol⁻¹. La concentration à la saturation dans 1 m³ (1000 l) d'air à la pression atmosphérique, soit à 750 mmHg, est alors :

$$\frac{46\,\text{g.mol}^{-1} \times 40\,\text{mmHg} \times 1000}{22,4\,\text{l.mol}^{-1} \times 750\,\text{mmHg}} = 110\,\text{g/m}^3$$

Dans les 1500 m³ d'air brassés par heure, il faut donc un débit saturé de :

$$\frac{1000 \times 1,125\,\text{g.h}^{-1}}{110\,\text{g}} = 10,23\,\text{l.h}^{-1}$$

Ce débit correspond au débit d'air à introduire dans le réservoir pour emporter l'éthanol.

Dans le cas d'espèces plus lourdes, le débit devra être plus important. Par exemple, pour une espèce ayant une pression de vapeur saturante de 5 mmHg à 20°C, en suivant le même raisonnement, on obtient un débit d'air à introduire dans le réservoir de l'ordre de 50 l.h⁻¹ et pour une pression de vapeur saturante de 0.5 mmHg, un débit de l'ordre de 500 l.h⁻¹.

L'augmentation du débit d'air en contact avec la surface 10 libre de la substance 2, au fur et à mesure que les espèces les plus volatiles sont diffusées et que le volume dans le réservoir diminue, assure la diffusion d'une plus grande partie des espèces à débit constant. Ainsi, en déterminant les espèces volatiles présentes dans la substance 2 ainsi que leur pression de vapeur saturante, il est possible d'ajuster avec précision les valeurs du débit d'air à introduire dans le réservoir pour obtenir une diffusion sensiblement constante.

Cet ajustement du débit peut être obtenu de différentes manières.

Par exemple, le réservoir 9 peut être placé sur une balance mesurant son poids. La mesure est transmise à un système de commande qui ajuste alors le débit d'air du générateur 7 pour obtenir une diffusion constante.

Selon une mise en oeuvre préférée, le générateur 7 assure un débit total constant et le débit arrivant dans le réservoir est ajusté. Cet ajustement est réalisé en augmentant la section ouverte du conduit 13 amenant le débit d'air dans le réservoir 9 au fur et à mesure que le volume de substance 2 restant dans le flacon 9 diminue.

Par « section ouverte », on entend ici la surface d'échange d'air du conduit 13 avec la chambre 11 de mélange, c'est-à-dire la surface du conduit 13 s'ouvrant dans la chambre 11 et par laquelle le débit d'air amené par le conduit 13 dans le flacon 9 s'échappe effectivement du conduit 13.

Selon un premier mode de réalisation, le conduit 13 d'amenée du débit d'air dans le flacon 9 présente, du côté de son extrémité 15 aval, une pluralité de conduits de longueurs différentes débouchant dans le flacon 9. Dès lors, dépendamment du volume de substance 2 (c'est-à-dire du niveau de la surface 10 libre dans le flacon 9), le nombre de conduits plongeant dans la substance varie. Ainsi, plus le niveau atteint est bas, plus le nombre de conduits immergés est petit et plus le nombre d'ouvertures à la surface 10 libre de la substance 2 est grand, augmentant ainsi la section ouverte totale et, ainsi, le débit d'air circulant dans la chambre 11 au-dessus de la substance.

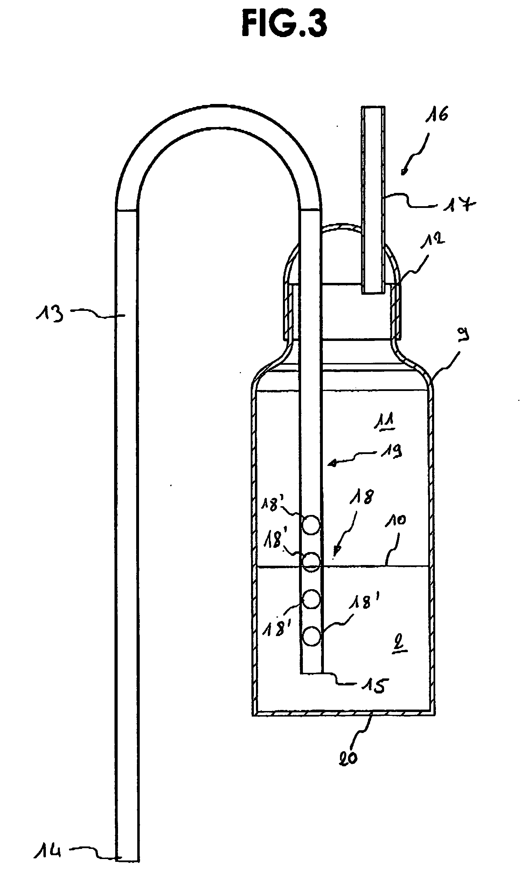

Selon un deuxième mode de réalisation, illustré sur les figures 3 à 6, le conduit 13 est muni d'au moins une ouverture 18 ménagée dans sa paroi latérale et débouchant dans le flacon 9.

Lorsque le flacon 9 est plein, l'extrémité 15 aval du conduit 13 est immergée dans la substance 2. Dans le cas où l'ouverture 18 latérale est également immergée, le débit d'air ne peut être amené dans le réservoir 9 et la diffusion des espèces les plus volatiles est assurée par l'évaporation naturelle des espèces les plus volatiles, jusqu'à ce que le niveau ait suffisamment diminué.

Lorsque l'ouverture 18 latérale est au moins en partie située au dessus du niveau de la surface 10 libre la substance 2, elle permet d'amener le débit d'air dans le réservoir 9. Ce débit étant insuffisant pour pénétrer dans la substance 2, l'air ne sort du conduit 13 dans le réservoir 9 que par cette ouverture 18. Ainsi, l'évaporation des espèces se faisant, le niveau de la surface 10 libre dans le réservoir 9 baisse, dévoilant graduellement l'ouverture 18, ce qui entraine l'augmentation de la section ouverte totale et du débit d'air dans le réservoir 9.

L'ouverture 18 latérale peut être réalisée de différentes manières.

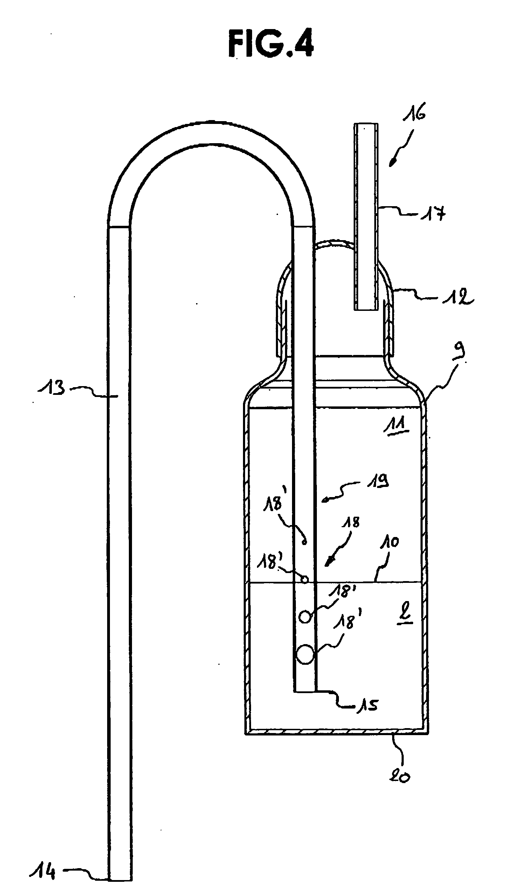

Par exemple, elle peut comprendre plusieurs ouvertures 18' latérales réparties sur la portion 19 du conduit s'étendant dans le flacon 9. Les ouvertures 18' peuvent être de forme quelconque. Par exemple, les ouvertures 18' sont conformées en cercle, de diamètre constant (figure 3) ou de diamètres croissants (figure 4) vers l'extrémité 15 aval. Dès lors, la section ouverte totale varie graduellement, les ouvertures 18' étant dévoilées les unes après les autres au fur et à mesure que le volume de la substance 2 diminue et que le niveau de la surface 10 libre baisse.

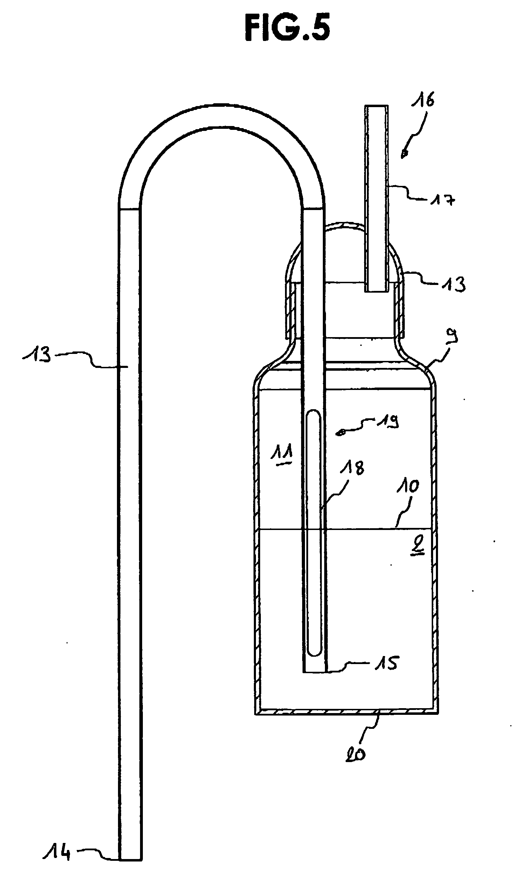

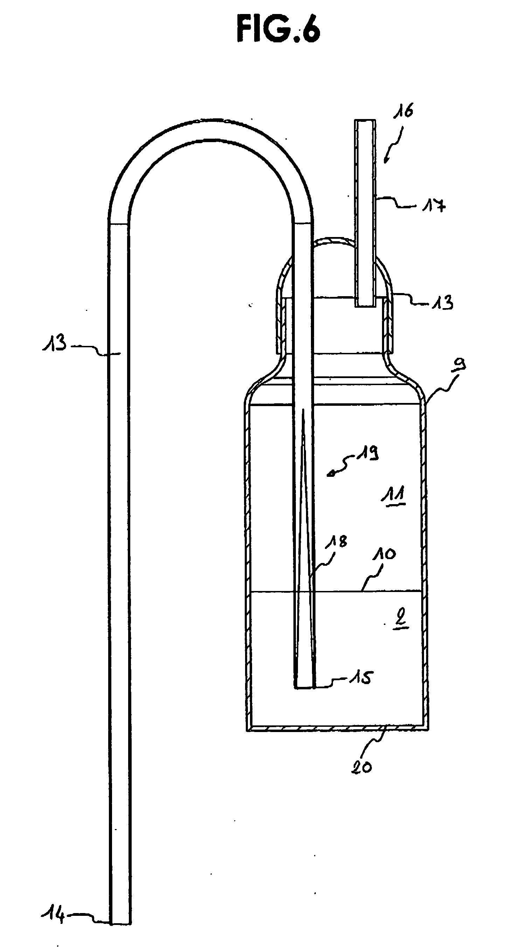

Une seule ouverture 18 continue peut également être réalisée, par exemple de forme allongée, s'étendant sur la portion 19 du conduit 13 dans le flacon 9. L'ouverture 18 unique peut se présenter par exemple sous la forme d'un trou oblong (figure 5), ou sous la forme d'une découpe s'évasant en direction de l'extrémité 15 aval (figure 6). La section ouverte totale, et donc le débit d'air dans le réservoir 9, augmentent de manière continue au fur et à mesure que le volume de la substance 2 diminue.

De préférence, l'extrémité 15 aval du conduit est ouverte, libre et dirigée vers le fond 20 du réservoir 9, de sorte que lorsque le volume dans le réservoir 9 diminue, il arrive que l'extrémité 15 aval se retrouve au dessus du niveau de la surface 10 libre la substance 2 et contribue à l'augmentation de la section ouverte totale du conduit 13.

De plus, l'ouverture latérale 18 apporte le débit d'air au plus près de la surface 10 libre de la substance 2, en particulier dans le cas d'une ouverture 18 continue, améliorant ainsi le brassage et le transport des espèces volatiles.

Afin d'optimiser l'évaporation, notamment des espèces les moins volatiles, un solide poreux peut en outre être introduit dans le flacon 9. Ce solide se présente par exemple sous la forme de particules sensiblement sphériques, telles que des billes, de sorte à laisser circuler l'air entre ces particules. Lorsqu'une certaine quantité de la substance liquide s'est évaporée, les particules poreuses ayant absorbé les espèces les moins volatiles, la surface d'échange totale, par laquelle l'évaporation s'effectue, se trouve augmentée. Ces particules peuvent être par exemple des boules d'un tissu absorbant ou des morceaux de pierre ponce. Par ailleurs, ces particules permettent d'absorber les huiles et autres composants non volatils de la substance 2, donnant alors à un utilisateur l'illusion d'une utilisation complète.

L'évaporation peut en outre être favorisée par un dispositif de chauffe, placé par exemple sous le flacon 9.

Par ailleurs, dans le cas d'un parfum, la nuance de fragrances entre les espèces de pression de vapeur saturante différente est suffisamment faible pour ne pas être discernable par une personne ayant un odorat ordinaire. Toutefois, afin d'homogénéiser les espèces diffusées dans un local, il est préférable de placer plusieurs réservoirs 9 de la substance sur la grille 8, chacun muni d'un conduit 13 amenant un débit d'air dans le flacon 9, ainsi que représenté sur la figure 1. Par exemple, un premier flacon 9 est d'abord mis en place. A partir d'une baisse de volume déterminée ou de niveau de la surface 10 libre, un deuxième flacon 9 est mis en place afin de mélanger les espèces les plus volatiles émanant de ce deuxième réservoir avec celles désormais plus lourdes émanant du premier flacon 9. D'autres flacons 9 peuvent également être mis en place successivement suivant le même principe.

Les flacons 9 successifs peuvent contenir la même substance 2 ou des substances différentes se mélangeant lors de la diffusion.

En prenant en compte les pressions de vapeur saturante des espèces d'une substance, le procédé de diffusion ainsi réalisé assure une diffusion plus complète que dans un procédé dans lequel les pressions de vapeur saturante sont négligées.

Par ailleurs, une mise en oeuvre simple dans laquelle la section ouverte totale du conduit 13 amenant le débit d'air dans le réservoir 9 varie en fonction du volume permet d'ajuster ce débit d'air en fonction d'espèces restantes dans le réservoir.

Cette mise en oeuvre est peu coûteuse et la substance 2 n'entre pas en contact avec des pièces ayant des fonctions mécaniques telles que le générateur 7 de débit d'air, qui peuvent être sensibles à la substance 2. Le dispositif nécessite ainsi moins d'entretien et a une durée de vie plus grande.

Le dispositif 1 de diffusion ainsi réalisé peut alors diffuser dans un local de grand volume une quantité maximale de substance 2 contenue dans un flacon 9.

**Revendications**

1. Procédé de diffusion d'une substance volatile dans un local, la substance issues de l'évaporation de la substance étant contenue dans un réservoir (9) comprenant des espèces présentant des pressions de vapeur saturante différentes et définissant en outre une chambre de mélange (11) située au-dessus de la substance (2), le procédé comprenant les étapes consistant à :

   - générer un débit d'air ;
   - introduire au moins une partie du débit d'air dans la chambre (11) de mélange contenant un mélange d'air et d'espèces volatiles issues de l'évaporation de la substance ;
   - diffuser le mélange dans l'atmosphère du local,

   ce procédé étant **caractérisé en ce que** le débit d'air introduit dans la chambre (11) de mélange est ajusté en fonction du volume de substance contenue dans le réservoir (9).

2. Procédé selon la revendication 1, dans lequel le débit d'air introduit dans la chambre de mélange est réajusté à la hausse avec la diminution du volume de substance dans le réservoir.

3. Dispositif de diffusion (1) d'une substance (2) volatile, ce dispositif (1) comportant :

   - un générateur (7) d'un débit d'air ; un réservoir (9) contenant la substance (2) et définissant en outre une chambre de mélange (11) située au-dessus de la substance (2) ;
   - la chambre de mélange (11) contenant un mélange d'air et d'espèces issues de l'évaporation de la substance ;
   - un conduit (13) d'amenée d'air mettant en communication le débit d'air avec la chambre (11), le conduit (13) présentant une surface (18) d'échange d'air avec la chambre (11) de mélange ;
   - une évacuation (16) mettant la chambre (11) de mélange en communication avec l'atmosphère,

   ce dispositif (1) étant **caractérisé en ce qu'**il comprend des moyens d'ajustement de la surface (18) d'échange en fonction du niveau de substance (2) dans le réservoir.

4. Dispositif selon la revendication 3, dans lequel la surface (18) d'échange comprend au moins une ouverture latérale ménagée dans le conduit (13) et débouchant au moins partiellement dans la chambre (11) de mélange.

5. Dispositif (1) selon la revendication 4, dans lequel le conduit (13) comprend plusieurs ouvertures (18') latérales dont l'une au moins débouche dans la chambre (11) de mélange.

6. Dispositif (1) selon la revendication 5, dans lequel les ouvertures (18') sont circulaires.

7. Dispositif (1) selon la revendication 6, dans lequel le diamètre des ouvertures (18') est croissant en direction d'une extrémité (15) libre aval du conduit (13).

8. Dispositif (1) selon la revendication 4, dans lequel le conduit (13) comprend une unique ouverture (18) latérale sous forme d'une découpe allongée.

9. Dispositif (1) selon la revendication 8, dans lequel ladite découpe va en s'évasant depuis la chambre (11) de mélange vers une extrémité (15) libre aval du conduit (13).

10. Dispositif (1) selon l'une des revendications 4 à 9, dans lequel le réservoir (9) comprend un solide poreux en contact avec la substance (2) pour absorber une quantité de la substance (2).

**Claims**

1. Method of diffusing a volatile substance in a room, the substance (2) being contained in a reservoir (9) comprising species having different saturated vapour pressures and further defining a mixing chamber (11) situated above the substance (2), the method comprising the steps consisting of:

   - generating a flow of air;

- introducing at least a part of the flow of air into the mixing chamber (11) containing a mixture of air and volatile species resulting from the evaporation of the substance;
- diffusing the mixture into the atmosphere of the room,

this method being **characterized in that** the flow of air introduced into the mixing chamber (11) is adjusted as a function of the volume of substance contained in the reservoir (9).

2. Method according to Claim 1, wherein the flow of air introduced into the mixing chamber is adjusted upward as the volume of substance in the reservoir decreases.

3. Device (1) for diffusing a volatile substance (2), this device (1) including:

- a generator (7) of a flow of air;
- a reservoir (9) containing the substance (2) and further defining a mixing chamber (11) situated above the substance (2);
- the mixing chamber (11) containing a mixture of air and species resulting from the evaporation of the substance;
- an air feed pipe (13) establishing communication between the flow of air and the chamber (11), the pipe (13) having an area (18) of exchange of air with the mixing chamber (11);
- a vent (16) establishing communication between the mixing chamber (11) and the atmosphere,

this device (1) being **characterized in that** it comprises means for adjusting the exchange area (18) as a function of the level of substance (2) in the reservoir.

4. Device according to Claim 3, wherein the exchange area (18) comprises at least one lateral opening formed in the pipe (13) and opening at least partially into the mixing chamber (11).

5. Device (1) according to Claim 4, wherein the pipe (13) comprises a plurality of lateral openings (18') at least one of which opens into the mixing chamber (11).

6. Device (1) according to Claim 5, wherein the openings (18') are circular.

7. Device (1) according to Claim 6, wherein the diameter of the openings (18') increases in the direction of a downstream free end (15) of the pipe (13).

8. Device (1) according to Claim 4, wherein the pipe (13) comprises a single lateral opening (18) in the form of an elongate cut-out.

9. Device (1) according to Claim 8, wherein said cut-out is flared from the mixing chamber (11) toward a downstream free end (15) of the pipe (13).

10. Device (1) according to any one of Claims 4 to 9, wherein the reservoir (9) comprises a porous solid in contact with the substance (2) to absorb a quantity of the substance (2).

**Patentansprüche**

1. Verfahren zum Diffundieren einer flüchtigen Substanz in einem Raum, wobei die Substanz (2) in einem Vorratsbehälter (9) enthalten ist, der Arten enthält, die verschiedene Sättigungsdampfdrücke aufweisen, und außerdem eine Mischkammer (11) definiert, die sich über der Substanz (2) befindet, wobei das Verfahren die folgenden Schritte umfasst, die darin bestehen:

- einen Luftdurchfluss zu erzeugen;
- wenigstens einen Teil des Luftdurchflusses in die Mischkammer (11) einzuleiten, die ein Gemisch aus Luft und flüchtigen Arten enthält, die aus der Verdampfung der Substanz hervorgehen;
- das Gemisch in die Atmosphäre des Raums zu diffundieren,

wobei das Verfahren **dadurch gekennzeichnet ist, dass** der in die Mischkammer (11) eingeleitete Luftdurchfluss als Funktion des Volumens der in dem Vorratsbehälter (9) enthaltenen Substanz eingestellt wird.

**2.** Verfahren nach Anspruch 1, wobei der in die Mischkammer eingeleitete Luftdurchfluss bei einer Abnahme des Volumens der Substanz in den Vorratsbehälter steigend neu eingestellt wird.

**3.** Vorrichtung (1) für die Diffusion einer flüchtigen Substanz (2), wobei diese Vorrichtung (1) Folgendes umfasst:

- einen Generator (7) für einen Luftdurchfluss;
- einen Vorratsbehälter (9), der die Substanz (2) enthält und außerdem eine Mischkammer (11) definiert, die sich über der Substanz (2) befindet;
- die Mischkammer (11), die ein Gemisch aus Luft und Arten, die aus der Verdampfung der Substanz hervorgehen, enthält;
- eine Luftzufuhrleitung (13), die eine Kommunikation zwischen dem Luftdurchfluss und der Kammer (11) herstellt, wobei die Leitung (13) eine Oberfläche (18) für den Austausch von Luft mit der Mischkammer (11) aufweist;
- einen Auslass (16), der eine Kommunikation zwischen der Mischkammer (11) und der Atmosphäre herstellt,

wobei diese Vorrichtung (1) **dadurch gekennzeichnet ist, dass** sie Mittel zum Einstellen der Austauschoberfläche (18) als Funktion des Pegels der Substanz (2) in dem Vorratsbehälter umfasst.

**4.** Vorrichtung nach Anspruch 3, wobei die Austauschoberfläche (18) wenigstens eine seitliche Öffnung aufweist, die in der Leitung (13) ausgebildet ist und wenigstens teilweise in die Mischkammer (11) mündet.

**5.** Vorrichtung (1) nach Anspruch 4, wobei die Leitung (13) mehrere seitliche Öffnungen (18') aufweist, wovon wenigstens eine in die Mischkammer (11) mündet.

**6.** Vorrichtung (1) nach Anspruch 5, wobei die Öffnungen (18') kreisförmig sind.

**7.** Vorrichtung (1) nach Anspruch 6, wobei der Durchmesser der Öffnungen (18') in Richtung eines freien stromabseitigen Endes (15) der Leitung (13) zunimmt.

**8.** Vorrichtung (1) nach Anspruch 4, wobei die Leitung (13) eine einzige seitliche Öffnung (18) in Form eines länglichen Ausschnitts aufweist.

**9.** Vorrichtung (1) nach Anspruch 8, wobei sich der Ausschnitt von der Mischkammer (11) zu dem freien stromabseitigen Ende (15) der Leitung (13) erweitert.

**10.** Vorrichtung (1) nach einem der Ansprüche 4 bis 9, wobei der Vorratsbehälter (9) einen porösen Feststoff enthält, der mit der Substanz (2) in Kontakt ist, um eine Menge der Substanz (2) zu absorbieren.

# FIG.1

# FIG.2

# FIG.3

# FIG.4

# FIG.5

# FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2002085542 A
- FR 2657019
- WO 20071128148 A
- EP 1252900 A
- FR 2797189